# EUROPEAN PATENT SPECIFICATION

(11) **EP 0 626 967 B1**
(45) Date of publication and mention of the grant of the patent: **08.10.2003**
(21) Application number: 93904228.9
(22) Date of filing: 18.02.1993
(51) Int. Cl.: C07D 493/04, C07D 491/044, C07D 301/03, C07B 53/00, C07F 13/00

(54) **PROCESS FOR PREPARING ENANTIOMERICALLY PURE FLUORINATED EPOXYCHROMANS**
VERFAHREN ZUR HERSTELLUNG VON ENANTIOMERREINEN FLUORIERTEN EPOXYCHROMANEN
PROCEDE DE PREPARATION D'EPOXYCHROMANES FLUORURES A PURETE ENANTIOMERE

(30) Priority: 20.02.1992 GB 9203596
(43) Date of publication of application: 07.12.1994
(73) Proprietor: SmithKline Beecham plc, Brentford, Middlesex TW8 9GS (GB)
(72) Inventor: BELL, David, SmithKline Beecham Pharmaceuticals, The Pinnacles, Harlow, Essex CM19 5AD (GB); FEDOULOFF, Michael, SmithKline Beecham, The Pinnacles, Harlow, Essex CM19 5AD (GB); TURNER, Gillian, SmithKline Beecham, The Pinnacles, Harlow, Essex CM19 5AD (GB)
(74) Representative: Rutter, Keith, Dr.
(86) International application number: GB9300344
(87) International publication number: WO93017026

(56) References cited:
- EP-A- 0 296 975
- EP-A- 0 376 524
- WO-A-91/14694
- TETRAHEDRON LETTERS. vol. 32, no. 38, 16 September 1991, OXFORD GB pages 5055 - 5058 NAM HO LEE ET AL 'Enantiomerically pure epoxychromans via asymmetric catalysis'

## Description

This invention relates to a novel process for preparing certain epoxides.

EP-A-0 376 524 describes certain compounds of formula (A): 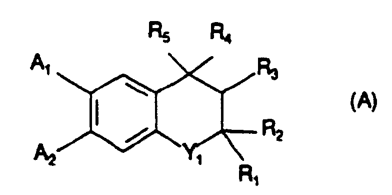 or, where appropriate, a pharmaceutically acceptable salt thereof, or a pharmaceutically acceptable solvate thereof
wherein:
one of A₁ or A₂ represents hydrogen and the other represents a group CF₃-Y- wherein Y represents -CF₂-, >C=O, or -CH(OH)-;
Y₁ represents -O-, -CH₂- or NR⁰ wherein R⁰ is hydrogen, alkyl or alkylcarbonyl;R₁ and R₂ independently represent hydrogen or alkyl; or
R₁ and R₂ together represent a C₂₋₇ polymethylene moiety;
R₃ represents hydrogen, hydroxy, alkoxy or acyloxy and R₄ is hydrogen or R₃ and R₄ together represent a bond;
R₅ represents either a moiety of formula (a): 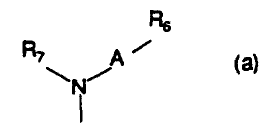 wherein A represents >C=X wherein X is O, S or NR₈ wherein R₈ represents CN, NO₂, COR₉ wherein R₉ is alkyl, amino, monoalkylamino, fluoroalkyl, phenyl or substituted phenyl or R₈ is SO₂R₉ wherein R₉ is as defined above, or A represents a bond;
   when A represents >C=X wherein X is O or S, then R₆ is hydrogen; alkyl optionally substituted by one or more groups or atoms selected from halogen, hydroxy, alkoxy, alkoxycarbonyl, carboxy or an ester or amide thereof, amino, monoalkylamino or dialkylamino; alkenyl; amino optionally substituted by an alkyl or alkenyl group or by an alkanoyl group optionally substituted by up to three halo atoms, by a phenyl group optionally substituted by alkyl, alkoxy or halogen; substituted or unsubstituted aryl or substituted or unsubstituted heteroaryl; and R₇ represents hydrogen or alkyl;
or R₆ and R₇ together represent a linking chain of formula -A₃-A₄-, A₃ being attached to the nitrogen atom of the moiety -N-A- and A₄ being attached to the group A on the said moiety, and wherein A₃ represents a substituted or unsubstituted methylene group, A₄ represents 2 or 3 linking members, one of the linking members optionally representing O, S or NR and the other linking members each independently representing a substituted or unsubstituted methylene group;
R represents hydrogen, alkyl, alkanoyl, phenyl C₁₋₄- alkyl, arylcarbonyl wherein the aryl group may be substituted or unsubstituted; or R is mono- or bi-cyclic- heteroarylcarbonyl;
when A represents >C=X wherein X represents NR₈, then R₆ represents -NH.R₁₀ wherein R₁₀ is hydrogen, alkyl, C₃₋₆ cycloalkyl, alkenyl or alkynyl and R₇ is hydrogen or alkyl; or R₇ and R₁₀ together represent C₂₋₄ polymethylene;
when A represents a bond, then R₆ and R₇ together with the nitrogen atom to which they are attached, form an unsaturated heterocyclic ring having 5 to 7 ring atoms, which ring atoms comprise up to 2 further nitrogen atoms and a carbon atom, the carbon atom being substituted with either an oxo group or a thioxo group, the remaining ring atoms being substituted or unsubstituted;
or R₅ represents a moiety of formula (b): 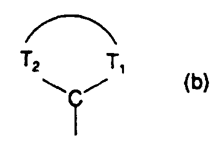 wherein T₁ represents >C-OH or N(O)ₙ wherein n is zero or 1 and T₂ together with C-T₁, when T₁ is >C-OH, represents an optionally substituted aryl group or T₂ together with CT₁, when T₁ is N(O)ₙ, represents an optionally substituted, N- heteroaryl group;
or R₅ represents a moiety of formula (c): 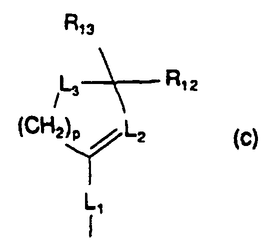 wherein L₁ represents O or NR₁₁ wherein R₁₁ represents hydrogen, alkyl, formyl, acetyl or hydroxymethyl, L₂ represents N or CL₄ wherein L₄ is hydrogen, halogen, formyl or hydroxymethyl, L₃ represents CH₂, O, S, >CHL₅ wherein L₅ is halogen or NL₆ wherein L₆ is hydrogen or alkyl and R₁₂ and R₁₃ each independently represent hydrogen or alkyl or R₁₂ together with R₁₃ represents oxo or thioxo; and p represents 1,2 or 3; which compounds are described as being useful as smooth muscle relaxants.

A useful intermediate in the preparation of compounds of formula (A) is an epoxide of formula (B): 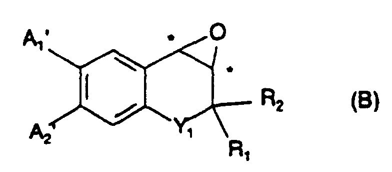 wherein A₁' and A₂' are A₁ or A₂ as defined in relation to formula (A), or groups convertible thereto and R₁, R₂ and Y₁ are as defined in relation to formula (A).

The carbon atoms marked with an asterisk (*) on formula (B) are chiral carbon atoms.

Previously, chemical methods for preparing the epoxide of formula (B) resulted in the formation of a racemic mixture of the epoxide: Any compound of formula (A) produced from such an epoxide would also be racemic and hence would need to be ressolved to yield an optionally pure product.

WO91/14694 describes certain catalysts which may be used for enantioselectivity epoxidizing prochiral olefins. However, there is no ,ention that such catalysts could be used to prepare substantially enantiomerically pure chiral epoxides of formula (B).

Surprisingly a new process has now been found which uses a particular catalysts from WO 91/14694 to produce the chiral epoxide of formula (B) in substantially enantiomerically pure form thus obviating the need for and resolution when preparing compounds of formula (A).

Accordingly, the present invention provides a process for preparing compounds of formula (B) (hereinbefore described) which process comprises reacting a compound of formula (C): 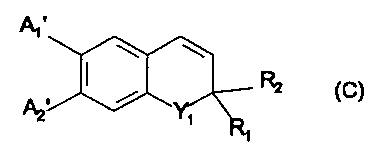 wherein A₁', A₂', R₁, R₂ and Y₁ are as defined in relation to formula (B) in the presence of an oxygen source and a chiral catalyst of formula (E): 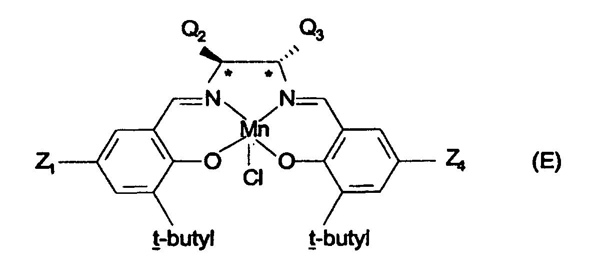 in which Z₁ and Z₄ are the same and are selected from the group consisting of methyl, t-butyl or methoxy and Q₂ and Q₃ are either both phenyl or together with the carbon atoms to which they are attached form an hexyl ring.

Most preferably, in catalysts of formula (E), Z₁ and Z₄ are both t-butyl and Q₂ and Q₃ together with the carbon atoms to which they are attached form an hexyl ring. Catalysts of formula (E) are a preferred sub-group of catalysts of formula (D) as defined in WO 91/14694: 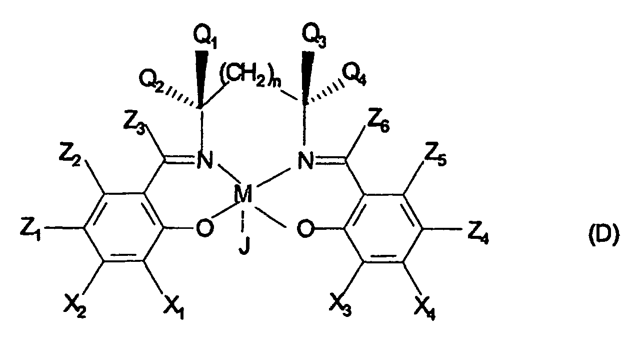 in which
M is a transition metal ion, J is an anion, and n is either 0, 1 or 2;at; at least one of X₁ or X₂ is selected from the group consisting of silyls, aryls, secondary alkyls and tertiary alkyls;and; and at least one of X₃ or X₄ is selected from the same group, Z₁, Z₂, Z₃, Z₄, Z₅ and Z₆ are independently selected from the group consisting of hydrogen, halides, alkyls, aryl groups, silyl groups, and alkyl groups bearing heteroatoms such as alkoxy and halide, also, at least one of Q₁, Q₂, Q₃ and Q₄ is selected from a first group consisting of H, CH₃, C₂H₅ and primary alkyls, furthermore, if Q₁ is selected from said first group, then Q₂ and Q₃ are selected from a second group consisting of aryl groups, heteroatom-bearing aromatic groups, secondary alkyls and tertiary alkyls; if Q₂ is selected from said first group, then Q₁ and Q₄ are selected from said second group; if Q₃ is selected from said first group, then Q₁ and Q₄ are selected from said second group; if Q₄ is selected from said first group, then Q₂ and Q₃ are selected from said second group, and thereafter when A₁' and/or A₂' are groups convertible to A₁ and A₂ respectively; converting A₁' and A₂' to A₁ and A₂ respectively.

It should be appreciated that the bond between M and J has varying degrees of ionic character depending on the anion used.

Preferred values for A1', A2', Y1, R1 and R2 in compounds of formula (C) are as defined in EP-A-0376 524.

Most preferably A1' is CF3CF2, A2' is hydrogen, Y1 is oxygen and R1 and R2 are both methyl.

Preferred values for M, J, n, X1, X2, X3, X4, Z1, Z2, Z3, Z4, Z5, Z6, Q1, Q2, Q3 and Q4 are as defined in WO/91/14694.

The reaction between the compound of formula (C) in the presence of an oxygen source and the chiral catalyst is suitably carried out using the procedures outlined in WO/91/14694 or procedures analogous thereto.

Suitably, the reaction is carried out as a two phase reaction with the compound of formula (C) and the chiral catalyst being dissolved in an inert solvent such as dichloromethane and the other phase being water with sodium hypochlorite added as the oxygen source, optionally in the presence of a buffer such as sodium dihydrogen phosphate, the pH being suitably adjusted to a pH of between 10 and 13, preferably between 10.5 and 12, most preferably between 11 and 11.5

The reaction is suitably carried out at reduced, ambient or elevated temperature, preferably at elevated temperature, such as greater than 30°C, preferably greater than 35°C, most preferably at 40°C to 45°C.

Suitably the mole ratio of chiral catalyst to compound of formula (C) is in the range of 0.01 to 10, preferably in the range of 1 to 5, most preferably in the range of 1 to 3.

Examples of groups A₁' and A₂' convertible to A₁ and A₂ and methods for their conversion are described in EP-A-0376524.

Preferred catalysts include the specific examples mentioned in WO 91/14694. Most preferably the chiral catalyst is (S,S)-[1,2-bis(3,5-di-tert-butylsalicylideneamino) cyclohexane]manganese (III) chloride as characterised on pages 33 and 34 of WO 91/14694.
It should be appreciated that the present invention specifically covers the preparation of all epoxide precursors to all specific examples in EP-A-0376 524 using the process hereinbefore described, especially the preparation of (3S,4S)-2,2-dimethyl-3,4-epoxy-6-pentafluoroethyl-2H-1-benzopyran.

Compounds of formula (C) are commercially available or may be prepared according to the procedures referred to or outlined in EP-A-0 376 524.

Chiral catalysts mentioned in WO/91/14694 may be prepared according to the procedures in WO 91/14694 or by procedures analogous to them.

The present applicationalso describesa process for preparing compounds of formula (A) (hereinbefore described) or, where appropriate, a pharmaceutically acceptable salt thereof, or a pharmaceutically acceptable solvate thereof which comprises reacting a compound of formula (C) (hereinbefore described) in the presence of an oxygen source and a chiral catalyst as defined in WO/91/14694 and thereafter converting the resulting compound of formula (B) to a compound of formula (A) or, where appropriate, a pharmaceutically acceptable salt thereof, or a pharmaceutically acceptable solvate thereof. Accordingly, in second aspect the present invention provides a process for preparing compounds of formula (A) 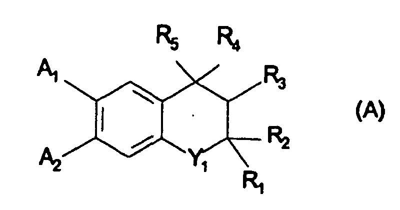 or, where appropriate, a pharmaceutically acceptable salt thereof, or a pharmaceutically acceptable solvate thereof
wherein:
one of A₁ or A₂ represents hydrogen and the other represents a group CF₃-Y- wherein Y represents -CF₂-, >C=O, or -CH(OH)-;
Y₁ represents -O-, -CH₂- or NR⁰ wherein R⁰ is hydrogen, alkyl or alkylcarbonyl; R₁ and R₂ independently represent hydrogen or alkyl; or R₁ and R₂ together represent a C₂₋₇ polymethylene moiety;
R₃ represents hydrogen, hydroxy, alkoxy or acyloxy and R₄ is hydrogen or R₃ and R₄ together represent a bond;
R₅ represents either a moiety of formula (a): 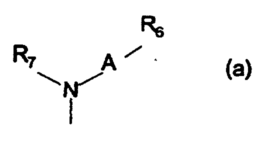 wherein A represents >C=X wherein X is O, S or NR₈ wherein R₈ represents CN, NO₂, COR₉ wherein R₉ is alkyl, amino, monoalkylamino, fluoroalkyl, phenyl or substituted phenyl or R₈ is SO₂R₉ wherein R₉ is as defined above, or A represents a bond;
   when A represents >C=X wherein X is O or S, then R₆ is hydrogen; alkyl optionally substituted by one or more groups or atoms selected from halogen, hydroxy, alkoxy, alkoxycarbonyl, carboxy or an ester or amide thereof, amino, monoalkylamino or dialkylamino; alkenyl; amino optionally substituted by an alkyl or alkenyl group or by an alkanoyl group optionally substituted by up to three halo atoms, by a phenyl group optionally substituted by alkyl, alkoxy or halogen; substituted or unsubstituted aryl or substituted or unsubstituted heteroaryl; and R₇ represents hydrogen or alkyl;
or R₆ and R₇ together represent a linking chain of formula -A₃-A₄-, A₃ being attached to the nitrogen atom of the moiety -N-A- and A₄ being attached to the group A on the said moiety, and wherein A₃ represents a substituted or unsubstituted methylene group, A₄ represents 2 or 3 linking members, one of the linking members optionally representing O, S or NR and the other linking members each independently representing a substituted or unsubstituted methylene group;
when A represents a bond, then R₆ and R₇ together with the nitrogen atom to which they are attached, form an unsaturated heterocyclic ring having 5 to 7 ring atoms, which ring atoms comprise up to 2 further nitrogen atoms and a carbon atom, the carbon atom being substituted with either an oxo group or a thioxo group, the remaining ring atoms being substituted or unsubstituted;
which process comprises preparing reacting a compound of formula (B), as defined in claim 1, according to the process of claim 1 and thereafter preparing a compound of formula (A) or, where appropriate a pharmaceutically acceptable salt thereof, or a pharmaceutically acceptable solvate thereof.

It should also be appreciated that the present process specifically encompasses the preparation of all the specific examples in EP-A-0376 524 by reacting appropriate compounds of formula (C) in the presence of an oxygen source and a chiral catalyst a defined in WO/91/14694 and thereafter converting the resulting compound of formula (B) to a compound of formula (A).

A preferred compound of formula (A) is (3S,4R)-3,4-dihydro-2,2-dimethyl-4-(2-oxopiperidin-1-yl)-6-penta-fluoroethyl-2H-1-benzopyran-3-ol, which is prepared by reacting (3S,4S)-2,2-dimethyl-3,4-epoxy-6-pentafluoroethyl-2H-1-benzopyran in the presence of an oxygen source and a chiral catalyst as defined in WO/91/14694 and thereafter converting the resulting epoxide compound to the compound of formula (A) as above.

Suitable methods for converting compounds of formula (B) to compounds of formula (A)include those mentioned in EP-A-0 376 524.

The following example illustrates the present invention.

### Example 1

### Preparation of (3S,4S)-2,2-Dimethyl-3,4-epoxy-6-pentafluoroethyl-2H-1-benzopyran

A titrated solution of sodium hypochlorite (2.5L) water (4.2L) and 0.05M sodium dihydrogen phosphate (16.0g) in 2.7L of deionised water was added to a 20L flanged flask and the mixture adjusted to pH 11.3 with a few drops of orthophosphoric acid. This solution was added to a solution of 2,2-dimethyl-6-pentafluoroethyl-2H-1- benzopyran (0.75kg) (which may be prepared according to the procedures outlined in EP-A-0376524) and (S,S)-[1,2-bis(3,5-di-tert-butylsalicylideneamino)cyclohexane]manganese (III) chloride (17.1g) (which may be prepared according to the procedures outlined in WO/91/14694 in dichloromethane (2.7L) in a 25L vessel and the mixture stirred at 42°C overnight. The solution was cooled to 20°C, dichloromethane (4.1L) was added, the mixture filtered through a bed of celite filter aid, washing the filter cake with dichloromethane (2.7L) and the phases separated. The aqueous phase was washed with dichloromethane (6.7L) and the combined organic phases were washed with brine (5.3L) and evaporated to dryness to give 800g of crude title product.

The crude epoxide was recrystallised from hot hexane (1.8L) to give a first crop of a white solid which was filtered off and washed with 3 x 0.5L portions of ice cold hexane. The solid was dried in vacuo at 50°C for 3 hours.

The mother liquors were concentrated to 1.1L and allowed to cool to 4°C for 3-4 hours. The second crop of solid was filtered off and the product washed with 2 X 0.3L of ice cold hexane. The solid was dried in vacuo at 50° for 3 hours.

Yield of first crop of title compound - 533g (67.2%).
Purity of first crop of title compound - 98.7% (determined by quantitative HPLC).

Enantiomeric excess of first crop of title compound - 99.7% (determined by quantitative chiral HPLC).

Yield of second crop of title compound - 36.5g (4.6%).
Purity of second crop of title compound - 97.1% (determined by quantitative HPLC.
Enantiomeric excess of second crop of title compound - 96.9% (determined by quantitative chiral HPLC).

## Claims

1. A process for preparing compounds of formula (B) 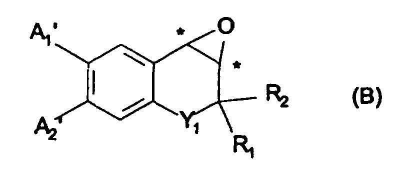 wherein:
one of A₁' or A₂' represents hydrogen and the other represents a group CF₃-Y- wherein Y represents -CF₂-, >C=O, or -CH(OH)-;
Y₁ represents -O-, -CH₂- or NR⁰ wherein R⁰ is hydrogen, alkyl or alkylcarbonyl; R₁ and
R₂ independently represent hydrogen or alkyl; or R₁ and R₂ together represent a C₂₋₇ polymethylene moiety;
which process comprises reacting a compound of formula (C): 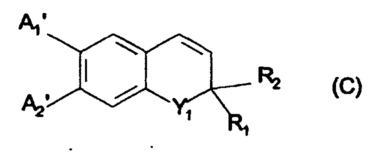 wherein the variables A₁', A₂', Y₁, R₁ and R₂ are as defined above, in the presence of an oxygen source and a chiral catalyst is of formula (E): 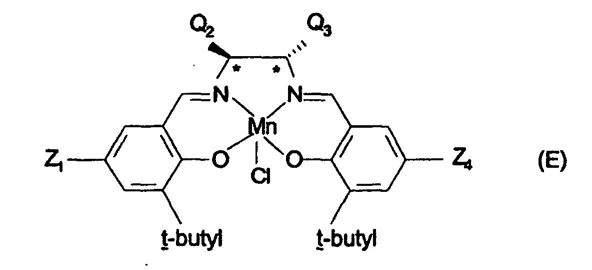 wherein Z₁ and Z₄ are the same and are selected from the group consisting of methyl, t-butyl or methoxy and Q₂ and Q₃ are either both phenyl or together with the carbon atoms to which they are attached form a hexyl ring.

2. A process according to claim 1 wherein in the compound of formula (B) A₁' is CF₃CF₂, A₂' is hydrogen, Y₁ is oxygen and R₁ and R₂ are both methyl.

3. A process according to claim 1 or 2 wherein the compound of formula (B) is 3S,4S-2,2-dimethyl-3,4-epoxy-6-pentafluoroethyl-2H-1-benzopyran.

4. A process according to claim 1 wherein Z₁ and Z₄ are both t-butyl and Q₂ and Q₃ together with the carbon atom to which they are attached form a hexyl group.

5. A process according to claim 4 wherein the chiral catalyst is (S,S)-[1,2-bis(3,5-di-tert-butylsalicylideneamino)cyclohexane]manganese (III) chloride.

6. A process for preparing compounds of formula (A) 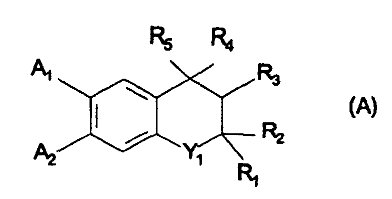 or, where appropriate, a pharmaceutically acceptable salt thereof, or a pharmaceutically acceptable solvate thereof
wherein:
one of A₁ or A₂ represents hydrogen and the other represents a group CF₃-Y- wherein Y represents -CF₂-, >C=O, or -CH(OH)-;
Y₁ represents -O-, -CH₂- or NR⁰ wherein R⁰ is hydrogen, alkyl or alkylcarbonyl; R₁ and R₂ independently represent hydrogen or alkyl; or R₁ and R₂ together represent a C₂₋₇ polymethylene moiety;
R₃ represents hydrogen, hydroxy, alkoxy or acyloxy and R₄ is hydrogen or R₃ and R₄ together represent a bond;
R₅ represents a moiety of formula (a): 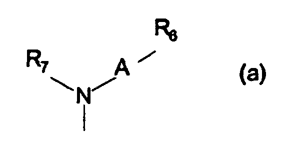 wherein A represents >C=X wherein X is O or S, or A represents a bond;
when A represents >C=X wherein X is O or S, then R₆ and R₇ together represent a linking chain of formula -A₃-A₄-, A₃ being attached to the nitrogen atom of the moiety -N-A- and A₄ being attached to the group A on the said moiety, and wherein A₃ represents a substituted or unsubstituted methylene group, A₄ represents 2 or 3 linking members, one of the linking members optionally representing O, S or NR and the other linking members each independently representing a substituted or unsubstituted methylene group;
R represents hydrogen, alkyl, alkanoyl, phenyl C₁₋₄- alkyl, arylcarbonyl wherein the aryl group may be substituted or unsubstituted; or R is mono- or bi-cyclic- heteroarylcarbonyl; ;
when A represents a bond, then R₆ and R₇ together with the nitrogen atom to which they are attached, form an unsaturated heterocyclic ring having 5 to 7 ring atoms, which ring atoms comprise up to 2 further nitrogen atoms and a carbon atom, the carbon atom being substituted with either an oxo group or a thioxo group, the remaining ring atoms being substituted or unsubstituted;
which comprises preparing a compound of formula (B), as defined in claim 1, according to the process of claim 1 and thereafter preparing a compound of formula (A) or, where appropriate a pharmaceutically acceptable salt thereof, or a pharmaceutically acceptable solvate thereof.

7. A process according to claim 6 in which the compound of formula (A) is (3S,4R)-3,4-dihydro-2,2-dimethyl-4-(2-oxopiperidin-1-yl)-6-penta-fluoroethyl-2H-1-benzopyran-3-ol.

## Patentansprüche

1. Verfahren zur Herstellung von Verbindungen der Formel (B) 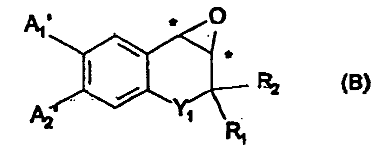 wobei:
einer der Reste A₁' oder A₂' ein Wasserstoffatom bedeutet und der andere einen Rest CF₃-Y- bedeutet, wobei Y einen Rest -CF₂-, >C=O oder -CH(OH)- bedeutet; Y₁ einen Rest -O-, -CH₂- oder NR⁰ bedeutet, wobei R⁰ ein Wasserstoffatom, einen Alkyl- oder Alkylcarbonylrest bedeutet; R₁ und R₂ unabhängig voneinander ein Wasserstoffatom oder einen Alkylrest bedeuten; oder R₁ und R₂ zusammen eine C₂₋₇-Polymethyleneinheit bedeuten;
wobei das Verfahren das Umsetzen einer Verbindung-der Formel (C): 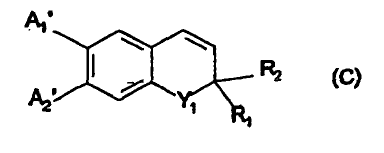 wobei die Variablen A₁', A₂', Y₁, R₁ und R₂ die vorstehende angegebene Bedeutung haben, in Gegenwart einer Sauerstoffquelle und eines chiralen Katalysators der Formel (E): 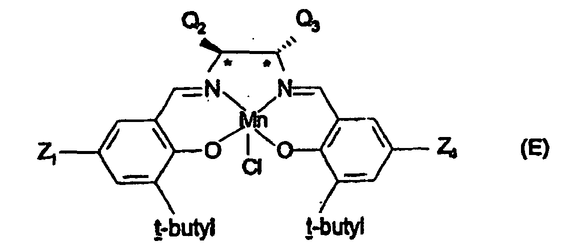 wobei Z₁ und Z₄ gleich und aus einer Methyl-, t-Butyl- oder Methoxygruppe ausgewählt sind und Q₂ und Q₃ entweder beide eine Phenylgruppe bedeuten oder zusammen mit den Kohlenstoffatomen, an die sie gebunden sind, einen Hexylring bilden, umfasst.

2. Verfahren gemäß Anspruch 1, wobei in der Verbindung der Formel (B) A₁' einen Rest CF₃CF₂ bedeutet, A₂' ein Wasserstoffatom bedeutet, Y₁ ein Sauerstoffatom bedeutet und R₁ und R₂ beide eine Methylgruppe bedeuten.

3. Verfahren gemäß Anspruch 1 oder 2, wobei die Verbindung der Formel (B) 3S,4S-2,2-Dimethyl-3,4-epoxy-6-pentafluorethyl-2H-1-benzopyran ist.

4. Verfahren gemäß Anspruch 1, wobei Z₁ und Z₄ beide eine t-Butylgruppe bedeuten und Q₂ und Q₃ zusammen mit den Kohlenstoffatomen, an die sie gebunden sind, einen Hexylring bilden.

5. Verfahren gemäß Anspruch 4, wobei der chirale Katalysator (S,S)-[1,2-Bis(3,5-di-*tert*butylsalicylidenamino)cyclohexan]mangan(III)chlorid ist.

6. Verfahren zur Herstellung von Verbindungen der Formel (A) 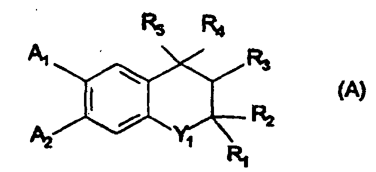 oder, sofern geeignet, eines pharmazeutisch verträglichen Salzes davon oder eines pharmazeutisch verträglichen Solvats davon, wobei:
einer der Reste A₁ oder A₂ ein Wasserstoffatom bedeutet und der andere einen Rest CF₃-Y- bedeutet, wobei Y einen Rest -CF₂-, >C=O oder -CH(OH)- bedeutet;
Y₁ einen Rest -O-, -CH₂- oder NR⁰ bedeutet, wobei R⁰ ein Wasserstoffatom, einen Alkyl- oder Alkylcarbonylrest bedeutet; R₁ und R₂ unabhängig voneinander ein Wasserstoffatom oder einen Alkylrest bedeuten; oder R₁ und R₂ zusammen eine C₂₋₇-Polymethyleneinheit bedeuten;
R₃ ein Wasserstoffatom, einen Hydroxy-, Alkoxy- oder Acyloxyrest bedeutet und R₄ ein Wasserstoffatom bedeutet oder R₃ und R₄ zusammen eine Bindung bedeuten;
R₅ eine Einheit der Formel (a): 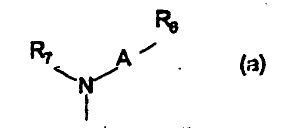 bedeutet, wobei A einen Rest >C=X bedeutet, wobei X gleich O oder S ist, oder A eine Bindung bedeutet;
falls A einen Rest >C=X bedeutet, in dem X gleich O oder S ist, dann bedeuten R₆ und R₇ zusammen eine verbindende Kette der Formel -A₃-A₄-, wobei A₃ an das Stickstoffatom der Einheit -N-A- gebunden ist und A₄ an den Rest A der Einheit gebunden ist, und wobei A₃ eine substituierte oder unsubstituierte Methylengruppe bedeutet, A₄ 2 oder 3 verbindende Glieder darstellt, wobei eines der verbindenden Glieder gegebenenfalls O, S oder NR bedeutet und die anderen verbindenden Glieder jeweils unabhängig voneinander eine substituierte oder unsubstituierte Methylengruppe bedeuten;
R ein Wasserstoffatom, einen Alkyl-, Alkanoyl-, Phenyl-C₁₋₄-alkyl-, Arylcarbonylrest bedeutet, wobei der Arylrest substituiert oder unsubstituiert sein kann; oder R ein monooder bicyclischer Heteroarylcarbonylrest ist;
falls A eine Bindung bedeutet, dann bilden R₆ und R₇ zusammen mit dem Stickstoffatom, an das sie gebunden sind, einen ungesättigten heterocyclischen Ring, der 5 bis 7 Ringatome aufweist, wobei die Ringatome bis zu 2 weitere Stickstoffatome und ein Kohlenstoffatom umfassen, wobei das Kohlenstoffatom entweder mit einer Oxooder Thioxogruppe substituiert ist, und wobei die restlichen Ringatome substituiert oder unsubstituiert sind;
welches die Herstellung einer Verbindung der Formel (B), wie in Anspruch 1 definiert, gemäß dem Verfahren des Anspruchs 1 und danach die Herstellung einer Verbindung der Formel (A) oder, sofern geeignet, eines pharmazeutisch verträglichen Salzes davon oder eines pharmazeutisch verträglichen Solvats davon, umfasst.

7. Verfahren gemäß Anspruch 6, wobei die Verbindung der Formel (A) (3S,4R)-3,4-Dihydro-2,2-dimethyl-4-(2-oxopiperidin-1-yl)-6-pentafluorethyl-2H-1-benzopyran-3-ol ist.

## Revendications

1. Procédé pour la préparation de composés de formule (B) 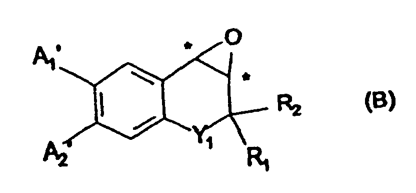 dans laquelle :
un des groupes A₁' et A₂' représente un atome d'hydrogène et l'autre représente un groupe CF₃-Y- dans lequel Y représente un groupe -CF₂-, >C=O ou -CH(OH)- ;
Y₁ représente un groupe -O-, -CH₂- ou NR⁰ dans lequel R⁰ représente un atome d'hydrogène, un groupe alkyle ou alkylcarbonyle ; R₁ et R₂ représentent indépendamment un atome d'hydrogène ou un groupe alkyle ; ou bien R₁ et R₂, conjointement, représentent un groupement polyméthylène en C₂ à C₇ ;
procédé qui comprend la réaction d'un composé de formule (C) : 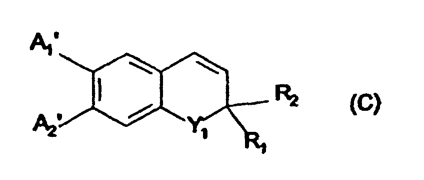 dans laquelle les variables A₁', A₂', Y₁, R₁ et R₂ répondent aux définitions précitées, en présence d'une source d'oxygène et d'un catalyseur chiral de formule (E) : 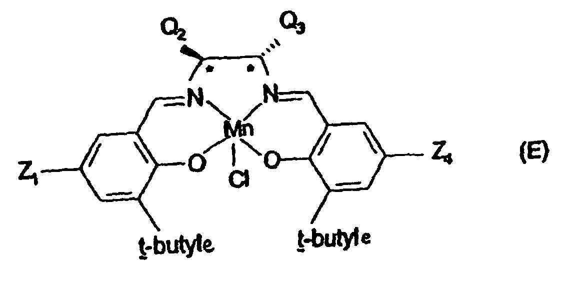 dans laquelle Z₁ et Z₄ sont identiques et sont choisis dans le groupe consistant en des groupes méthyle, tertio-butyle et méthoxy, et Q₂ et Q₃ représentent l'un et l'autre un groupe phényle ou bien, conjointement avec les atomes de carbone auxquels ils sont fixés, forment un noyau hexyle.

2. Procédé suivant la revendication 1, dans lequel, dans le composé de formule (B), A₁' représente un groupe CF₃CF₂, A₂' représente un atome d'hydrogène, Y₁ représente un atome d'oxygène et R₁ et R₂ représentent l'un et l'autre un groupe méthyle.

3. Procédé suivant la revendication 1 ou 2, dans lequel le composé de formule (B) est le 3S, 4S-2,2-diméthyl-3,4-époxy-6-pentafluoréthyl-2H-1-benzopyranne.

4. Procédé suivant la revendication 1, dans lequel Z₁ et Z₄ représentent l'un et l'autre un groupe tertio-butyle et Q₂ et Q₃, conjointement avec l'atome de carbone auquel ils sont fixés, forment un groupe hexyle.

5. Procédé suivant la revendication 4, dans lequel le catalyseur chiral est le chlorure de (S,S)-[1,2-bis-(3,5-di-tertio-butylsalicylidènamino)-cyclohexane]-manganèse (III).

6. Procédé pour la préparation de composés de formule (A) 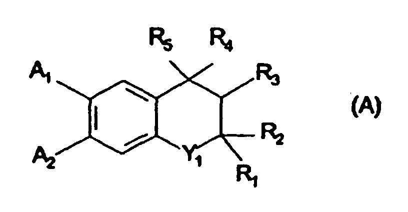 ou, lorsque cela est approprié, d'un de leurs sels pharmaceutiquement acceptables, ou d'un de leurs produits de solvatation pharmaceutiquement acceptables,
formule dans laquelle :
un des groupes A₁ et A₂ représente un atome d'hydrogène et l'autre représente un groupe CF₃-Y- dans lequel Y représente un groupe -CF₂-, >C=O, ou CH(OH)- ;
Y₁ représente un groupe -O-, -CH₂- ou NR⁰ dans lequel R⁰ représente un atome d'hydrogène, un groupe alkyle ou alkylcarbonyle ; R₁ et R₂ représentent indépendamment un atome d'hydrogène ou un groupe alkyle ; ou bien R₁ et R₂, conjointement, représentent un groupement polyméthylène en C₂ à C₇ ;
R₃ représente un atome d'hydrogène, un groupe hydroxy, alkoxy ou acyloxy et R₄ représente un atome d'hydrogène, ou bien R₃ et R₄, conjointement, représentent une liaison ;
R₅ représente un groupement de formule (a) : 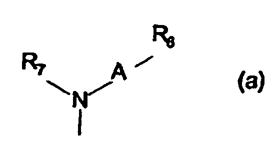 dans laquelle A représente un groupe >C=X dans lequel X représente O ou S, ou bien A représente une liaison ;
lorsque A représente un groupe >C=X dans lequel X représente O ou S, alors R₆ et R₇, conjointement, représentent une chaîne de liaison de formule -A₃-A₄-, A₃ étant fixé à l'atome d'azote du groupement -N-A- et A₄ étant fixé au groupe A sur ledit groupement, et A₃ représentant un groupe méthylène substitué ou non substitué, A₄ représentant deux ou trois membres de liaison, un des membres de liaison représentant facultativement un groupe O, S ou NR et les autres membres de liaison représentant chacun indépendamment un groupe méthylène substitué ou non substitué ;
R représente un atome d'hydrogène, un groupe alkyle, alcanoyle, phényl-(alkyle en C₁ à C₄), arylcarbonyle dans lequel le groupe aryle peut être substitué ou non substitué ; ou bien R représente un groupe hétéroarylcarbonyle mono- ou bicyclique ;
lorsque A représente une liaison, alors R₆ et R₇, conjointement avec l'atome d'azote auquel ils sont fixés, forment un noyau hétérocyclique insaturé comprenant 5 à 7 atomes dans le noyau, atomes du noyau qui comprennent jusqu'à 2 atomes d'azote supplémentaires et un atome de carbone, l'atome de carbone étant substitué avec un groupe oxo ou un groupe thioxo, les atomes restants du noyau étant substitués ou non substitués ;
qui comprend la préparation d'un composé de formule (B), répondant à la définition figurant dans la revendication 1, par le procédé suivant la revendication 1, et ensuite la préparation d'un composé de formule (A) ou, lorsque cela est approprié, d'un de ses sels pharmaceutiquement acceptables ou d'un de ses produits de solvatation pharmaceutiquement acceptables.

7. Procédé suivant la revendication 6, dans lequel le composé de formule (A) est (3S, 4R)-3,4-dihydro-2,2-diméthyl-4-(2-oxopipéridine-1-yl)-6-penta-fluoréthyl-2H-1-benzopyranne-3-ol.
